# EUROPÄISCHE PATENTANMELDUNG

(11) **EP 4 620 500 A1**
(43) Veröffentlichungstag der Anmeldung: **24.09.2025**
(21) Anmeldenummer: 25164076.9
(22) Anmeldetag: 17.03.2025
(51) Int. Cl.: A61M 5/315, A61M 5/31

(54) **DOSIERHILFE FÜR EINE SPRITZE UND SET MIT SPRITZE UND DOSIERHILFE**

(30) Priorität: 18.03.2024 DE 102024107696
(71) Anmelder: Albomed GmbH, 90592 Schwarzenbruck (DE)
(72) Erfinder: REESE, Sven, 90952 Schwarzenbruck (DE); BORKENSTEIN, Andreas, 8010 Graz (AT)
(74) Vertreter: Lichti - Patentanwälte Partnerschaft mbB

(57) **Zusammenfassung**

Zum Einstellen des von einer Spritze (1) abgebbaren Volumens (17) wird eine Dosierhilfe (13) mit einem Distanzstück (14) vorgeschlagen, die mit der Spritze (1) lösbar verbindbar ist, wobei das Distanzstück (14) einen den distalen Vorschub des Kolbens (3) begrenzenden Anschlag (15) für den Kolben (3) bildet und über mindestens eine Sollbruchstelle (18) mit einem manuell brechbaren Teilstück (14') verbunden ist.

## Beschreibung

Die Erfindung bezieht sich auf eine Dosierhilfe für eine Spritze und ein Set umfassend eine Spritze und die Dosierhilfe.

Eine Spritze ist ein medizinisches Instrument, das zum Verabreichen von Medikamenten, Nähr- und Infusionslösungen (als Injektion bzw. Infusion), zum Entnehmen von Körperflüssigkeiten (Punktion) oder -gewebe (Biopsie) sowie zum Spülen (beispielsweise von Wunden) verwendet wird. Eine Spritze umfasst einen Zylinder mit einem Hohlraum, einen in dem Zylinder axial beweglichen Kolben, der von einer Kolbenstange manuell betätigbar ist, und eine konus- oder zylinderförmige Düse am distalen Ende des Zylinders, an die eine Hohlnadel (Kanüle) oder ein Schlauch angeschlossen werden kann.

Beim Injizieren von Medikamenten, Nähr- und Infusionslösungen mit einer Spritze ist es wichtig, dass ein gewünschtes, vorbestimmtes Volumen, das in diesem Zusammenhang auch Dosis oder Dosiervolumen genannt wird, exakt verabreicht wird. Aus diesem Grund hat eine Spritze nach dem Stand der Technik einen durchsichtigen oder durchscheinenden Zylinder oder ein in den Zylinder eingelassenes Sichtfenster sowie eine Skala auf der Außenfläche des Zylinders, die die Menge der von der Spritze abgegebenen Flüssigkeit anzeigt, wenn der Kolben aus der mittels der Skala angezeigten Messposition ganz in den Zylinder eingedrückt wird. Das Ablesen der Skala erfolgt an dem distalen Ende des Kolbens, wobei die Skala auch das in der Spritze beim vollständigen Vorschieben des Kolbens in der Spritze verbleibende Restvolumen, beispielsweise in der Düse, berücksichtigt. Bei einer solchen herkömmlichen Spritze ist es für den Benutzer lästig, auf die Skala der benutzten Spritze schauen zu müssen. Ferner können Ablesefehler auftreten. Außerdem ist es schwer, beim Füllen der Spritze den Kolben genau an einer vorbestimmten Stelle der Skala der Spritze anzuhalten, sodass die gewünschte Injektionsmenge nicht oder nicht hinreichend genau eingehalten wird. Die Skala ist zumeist in äquidistante Abstände und demzufolge in entsprechende fixe Einheiten (Volumen, Dosen) unterteilt.

In vielen Anwendungsfällen sind die Benutzerfreundlichkeit und/oder die Genauigkeit der bekannten Spritzen nicht ausreichend, insbesondere dann nicht, wenn es entscheidend auf die Applikation eines exakten Dosiervolumens ankommt, zumal Dosierungen von Medikamenten oft an die körperlichen und/oder anatomischen Gegebenheiten eines Patienten angepasst werden müsse, was in vielen Fällen nicht mit den bekannten Spritzen exakt und wiederholgenau zu realisieren ist.

Dies gilt insbesondere für den Bereich der Ophthalmologie, beispielsweise bei einer intravitrealen Injektion (abgekürzt IVI, auch intravitreale operative Medikamentenapplikation (IVOM) genannt). Das Volumen einer intravitrealen Injektion, d. h. einer Injektion in den Glaskörper des Auges, beträgt typischerweise 0,05 ml bis 0,2 ml und es ist bekannt, dass das Komplikationsprofil von dem injizierten Volumen abhängt. Eine der möglichen Komplikationen ist ein Anstieg des Augeninnendrucks, der durch ein zu großes injiziertes Medikamentenvolumen oder dessen intraokuläre Ausdehnung bedingt sein kann. Ferner ist bekannt, dass bei größeren Injektionsvolumen von mehr als 0,2 ml das Risiko für eine augendruckbedingte retinale Perfusionsstörung signifikant zunimmt.

Gerade bei intravitrealen Injektionen müssen sehr kleine Volumina mit hoher Präzision appliziert werden. Um eine vorgegebene Dosis einzuhalten, werden grundsätzlich mit dem zu applizierenden Mittel vorgefüllte Fertigspritzen zu verwenden. Diese sind jedoch nur in vorgegeben fixen Dosen verfügbar und nur grob auf das Anwendungsgebiet, in der Regel jedoch nicht auf den Patienten. Eine Feinabstimmung oder Veränderung des Applikationsvolumen hinsichtlich des Patienten ist damit nicht möglich.

Beispielsweise ist aus der US 2004 / 0 162 528 A ein Gehäuseteil bekannt, das auf den Spritzenzylinder einer Spritze aufgesteckt wird und mittels eines in mehreren diskret definierten axialen Positionen anordenbaren Vorsprungs den Hub des Spritzenkolbens begrenzt. Hierfür muss jedoch das Gehäuseteil aufwändig von der Spritze entfernt und in einer neuen axialen Position relativ zur Spritze mit dieser verbunden werden.

Ausgehend von diesem Stand der Technik liegt der vorliegenden Erfindung die Aufgabe zugrunde, eine Dosierhilfe zu entwickeln, die die Nachteile aus dem Stand der Technik beseitigt und eine bessere Bedienbarkeit ermöglicht.

Diese Aufgabe wird durch eine Dosierhilfe mit den Merkmalen des Anspruchs 1 und ein Set mit den Merkmalen des Anspruchs 12 gelöst. Die erfindungsgemäße Dosierhilfe für eine Spritze, die einen Zylinder und einen Kolben umfasst, der mittels einer Kolbenstange in dem Zylinder axial beweglich ist, ist mit der Spritze lösbar verbindbar und weist in einem proximalen Bereich mindestens ein sich nach proximal erstreckendes Distanzstück auf, das einem Anschlag für den Kolben entspricht, wobei das Distanzstück über eine Sollbruchstelle mit einem Teilstück derart verbunden ist, dass das Teilstück manuell abbrechbar ist. Das erfindungsgemäße Set weist eine Spritze, insbesondere eine Spritze für die intravitreale Injektion, auf und die erfindungsgemäße Dosierhilfe, wobei die Dosierhilfe mit der Spritze lösbar verbindbar, insbesondere lösbar verbunden, ist.

Die Erfindung basiert auf dem Grundgedanken, dass durch das über die Sollbruchstelle mit dem Distanzstück verbundene, manuell abbrechbare Teilstück mehrere Applikationsvolumina einstellbar sind. Beispielsweise kann ein erstes Applikationsvolumen durch den axialen Hub zwischen der Ausgangsstellung des Kolbens einerseits und der axialen Position des Kolbens bei dem durch das Teilstück entsprechenden Anschlag andererseits definiert sein. Ein zweites Applikationsvolumen kann definiert sein durch einerseits der letztgenannten axialen Position des Kolbens und andererseits jener axialen Position des Kolbens, die sich ergibt, wenn das Teilstück abgebrochen ist und der Kolben bis zum nächsten Anschlag, der beispielsweise durch das Distanzstück selbst ausgebildet ist, weiter nach distal geschoben wird. Ein drittes Injektionsvolumen kann definiert sein durch den axialen Hub zwischen dieser Position des Kolbens und dessen Position, wenn der Kolben maximal nach distal bewegt ist und er sich in Anlage mit dem Zylinder der Spritze befindet. Insbesondere durch die axiale Länge des abbrechbaren Teilstücks kann ein gewünschtes Injektionsvolumen einstellbar sein. Ein weiteres Injektionsvolumen kann definiert sein durch den axialen Hub zwischen der Position des Kolbens, wenn er sich in Anlage mit dem, nicht abgebrochenen Teilstück der Dosierhilfe befindet, und jener Position, wenn sich der Kolben bei von der Spritze entfernter Dosierhilfe im Anschlag mit dem Zylinder der Spritze befindet. Dieses Injektionsvolumen kann größer sein als das oben genannte dritte Injektionsvolumen. Im Sinne der Erfindung kann auch die Dosierhilfe selbst einen Anschlag für den Kolben bilden.

Durch das manuell abbrechbare Teilstück wird eine präzise Applikation des in der Spritze befindlichen Mittels in einer für den Benutzer einfachen Weise möglich, da eine die Aufmerksamkeit des Benutzers beeinträchtigende Kontrolle einer Skala während des Applikationsvorgangs nicht mehr erforderlich ist. Die Benutzerfreundlichkeit bei der Verwendung der erfindungsgemäßen Dosierhilfe ist maßgeblich verbessert. Entsprechendes gilt für das erfindungsgemäße Set. Aufgrund der lösbaren Verbindung zwischen der Spritze und der Dosierhilfe kann letztere wiederverwendbar sein.

Den Kern der Erfindung vereinfachend zusammengefasst weist die abnehmbar bzw. anclipbar an einer Spritze befestigbare erfindungsgemäße Dosierhilfe, die zum Einstellen eines von der Spritze abgebbaren, vorbestimmt einstellbaren Volumens einer Flüssigkeit vorgesehen und ausgebildet ist, indem die Länge ihres Distanzstücks und/oder des Teilstücks so eingestellt wird, dass sie den Verfahrweg der Kolbenstange beim Einschieben des Kolbens in den Zylinder mechanisch begrenzt, so dass ein für das spätere Verabreichen gewünschtes Volumen, inklusive Totvolumen der Spritze, im Zylinder der Spritze verbleibt, und die an der Spritze befestigt wird, ein oder mehrere Teilstücke auf, mit der bzw. mit denen die Länge des den Vorschub des Kolbens begrenzenden Distanzstücks und/oder Teilstücks, wenn es auf die Spritze aufgesetzt ist, durch manuelles (das heißt ohne Verwenden eines Werkzeugs) Abbrechen eines oder mehrerer Teilstücke verkürzbar und somit das von der Spritze abgebbare Volumen mittels des nach dem Abbrechen verbleibenden Distanzstücks und gegebenenfalls eines oder mehrerer damit verbundener Teilstücke variabel vorbestimmt einstellbar ist. Die Dosierhilfe ist ein an einer Spritze anbringbares Teil, mit dem das während eines Applikationsvorgangs mit der Spritze zu applizierende Volumen genau auf einen vorbestimmten Wert eingestellt und beschränkt wird, der ein vordefiniertes Teilvolumen des maximal mit der Spritze abgebbaren Volumens ist, und wobei mittels der Dosierhilfe das zu applizierende Volumen aufgrund des über Sollbruchstellen mit dem Distanzstück verbundenen Teilstücks auf einfache und genaue Weise auf mehrere verschiedene, vordefinierte Werte eingestellt werden kann. Die Dosierhilfe mit dem Distanzstück und dem Teilstück zum Einstellen des von einer Spritze abgebbaren Volumens ist manuell auf die Spritze aufsetzbar und manuell von der Spritze abnehmbar, wobei das Teilstück einen den distalen Vorschub des Kolbens begrenzenden Anschlag für den Kolben bildet und mindestens eine Sollbruchstelle aufweist, an der es manuell brechbar ist.

Im Sinne der Erfindung weist eine axiale Richtung in Längsrichtung der Spritze, wobei eine proximale Richtung zum Benutzer und eine distale Richtung zum Patienten weist. Im Sinne dreidimensionaler Zylinderkoordinaten ist eine radiale Richtung senkrecht zur axialen Richtung angeordnet.

Das Material, aus dem die Dosierhilfe, insbesondere das Distanzstück und/oder das abbrechbare Teilstück besteht, kann jedes feste, ausreichend starre Material, insbesondere Kunststoff oder Metall sein. Je nach Beschaffenheit kann es erforderlichenfalls desinfiziert oder sterilisiert oder anderweitig im medizinischen Bereich aufbereitet sein, z. B. mittels Ethylenoxid-Sterilisation, Gamma-Strahlung oder Dampf, insbesondere mittels eines Autoklaven, oder mittels anderer Verfahren zur Desinfektion, Sterilisation und/oder Aufbereitung im medizinischen Bereich.

Die erfindungsgemäße Dosierhilfe kann wie folgt angewendet werden. Gemäß einer ersten Anwendungsmöglichkeit wird die Dosierhilfe auf ein gewünschtes, von einer Spritze abgebbares und mittels der Dosierhilfe vorbestimmt einstellbares Volumen einer Flüssigkeit eingestellt. Ist dieses gewünschte Volumen kleiner als es der Länge des Distanzstücks mitsamt dem Teilstück entspricht, also ohne dass das Teilstück abgebrochen wurde, wird hierzu an einer Sollbruchstelle, deren Lage derart ist, dass die zugehörige axiale Länge des Teilstücks dem gewünschten Volumen entspricht, abgebrochen. Die Dosierhilfe wird also durch Abbrechen des Teilstücks an einer Sollbruchstelle auf das gewünschte Maß gebracht. Danach wird die Dosierhilfe an der Spritze angebracht und fixiert. Wenn das gewünschte Volumen der maximalen Länge des Distanzstücks einschließlich des Teilstücks entspricht, also ohne dass es an der Sollbruchstelle abgebrochen ist, wird die Dosierhilfe entsprechend ohne Abbrechen des Teilstücks verwendet. Gemäß einer zweiten, alternativen Anwendungsmöglichkeit ist die erläuterte Reihenfolge umgekehrt, das heißt, zuerst wird die Dosierhilfe an der Spritze angebracht und fixiert und erst danach wird das Teilstück an der zu dem gewünschten Volumen passenden Sollbruchstelle abgebrochen.

Entweder bevor oder nachdem das Teilstück an der Sollbruchstelle abgebrochen wurde, kann die Spritze in der üblichen Weise blasenfrei mit dem zu applizierenden Mittel aufgezogen werden. Hierzu wird durch Zurückziehen des Kolbens die Spritze mit Flüssigkeit aufgezogen, entweder vollständig oder zumindest mit einem Volumen, das größer ist als das später zu applizierende Volumen, und danach durch senkrechtes Halten der Spritze nach oben und gleichzeitiges Hineindrücken des Kolbens, bis keine Luft, sondern Flüssigkeit aus der Düse der Spritze bzw. einer auf sie aufgesetzten Nadel austritt, die in der Spritze enthaltene Luft entfernt. Das zu diesem Zeitpunkt in der Spritze aufgezogene Volumen der Flüssigkeit ist größer als das mit der Spritze zu applizierende Volumen, das mittels der Dosierhilfe eingestellt wird.

Die Dosierhilfe wird nun mit der Spritze verbunden sowie, sofern das nicht zuvor erfolgte, durch Abbrechen des Teilstücks an der Sollbruchstelle auf das gewünschte, dem zu applizierenden Volumen entsprechende Maß gebracht. Dann wird der Kolben durch Hineindrücken der Kolbenstange soweit in den Zylinder geschoben, bis die auf die Spritze aufgesetzte Dosierhilfe mittels des von dem Distanzstück gebildeten Anschlags den weiteren distalen Vorschub des Kolbens begrenzt. Der Kolben ist also soweit in den Zylinder hineingedrückt, bis der von der Dosierhilfe mit seinem Distanzstück gebildete Anschlag das weitere Vorschieben des Kolbens verhindert. Das gewünschte Volumen der mittels der Spritze zu applizierende Flüssigkeit ist nun durch die Dosierhilfe an der Spritze eingestellt. Danach wird die Dosierhilfe von der Spritze abgenommen, wobei darauf zu achten ist, dass der Kolben nicht axial bewegt wird, um zu vermeiden, dass Flüssigkeit ausgestoßen oder Luft eingesaugt wird.

Die Spritze ist dann einsatzbereit, um mit ihr das gewünschte und mittels der Dosierhilfe vorbestimmt eingestellte Volumen abzugeben. Hierzu wird die Düse an das entsprechende Gegenstück angesetzt oder die Nadel an der Düse in das zu spritzende Objekt eingeführt und dann der Kolben soweit wie möglich in den Zylinder hineingedrückt, sodass sich die Spritze bis auf kleine, darin verbleibende Reste entleert.

Vorzugsweise weist die Dosierhilfe mindestens zwei axial hintereinander angeordnete Teilstücke auf, die jeweils über Sollbruchstellen miteinander derart verbunden sind, dass die Teilstücke manuell abbrechbar sind. Dadurch können mehrere, insbesondere während eines einzigen Eingriffs zu applizierende Dosierungen durch die Wahl der axialen Länge der Teilstücke einstellbar sein.

In einer Weiterentwicklung der Erfindung kann die Dosierhilfe zwischen einem und zehn Sollbruchstellen, bevorzugt zwischen zwei und acht Sollbruchstellen und besonders bevorzugt zwischen drei und sechs Sollbruchstellen oder Teilstücke aufweisen, um weitere zu applizierende Dosierungen bei einer einzigen Spritze zu ermöglichen. Gemäß einem weiteren vorteilhaften Merkmal kann vorgesehen sein, dass das proximale Ende des Teilstücks, insbesondere des distalen Teilstücks, der Dosierhilfe eine Anschlagsfläche aufweist, die derart ausgebildet ist, dass sie das axiale Bewegung des Kolbens nach distal begrenzt, wenn die Dosierhilfe auf die Spritze aufgesetzt ist und das distale Ende eines an der Kolbenstange angeordneten Kolbenkopfes an der Anschlagsfläche anliegt. Auf diese Weise begrenzt das Teilstück der auf die Spritze aufgesetzten Dosierhilfe den distalen Vorschub des Kolbens auf die Position, in der Kolbenkopf an dem Teilstück anliegt.

Vorzugsweise ist die Dosierhilfe derart ausgebildet, dass sie mit dem Zylinder der Spritze verbindbar ist. Auf diese Weise kann das mit der Spritze zu applizierende Volumen genau und reproduzierbar festgelegt werden. Der proximale Endbereich der Dosierhilfe, insbesondere des Distanzstücks und/oder des Teilstücks, kann dabei einen Anschlag für die distale Bewegung des Kolbens bilden.

Gemäß einer vorteilhaften weiteren Ausgestaltung kann dabei zum genau und reproduzierbaren Festlegen des mit der Spritze zu applizierenden Volumens vorgesehen sein, dass die Dosierhilfe eine teilkreisförmige Vertiefung, oder einen teilkreisförmigen Schlitz, aufweist, die bzw. der auf einen radialen Vorsprung am proximalen Ende des Zylinders aufsteckbar ist, wobei insbesondere das mindestens eine Distanzstück und/oder das mindestens eine Teilstück in einer vordefinierten axialen Relativposition mit der Spritze anordenbar ist. Eine solche teilkreisförmige Vertiefung kann auch als Ringnut bezeichnet werden. Die Vertiefung bzw. der Schlitz und der Vorsprung bilden vorzugsweise eine manuell lösbare Rastverbindung zwischen dem Zylinder und der darauf aufgesetzten Dosierhilfe. Der Vorsprung kann ein Teil einer herkömmlichen Spritze sein, insbesondere eine Fingerauflage der Spritze, die beispielsweise als sich in radialer Richtung erstreckende umlaufende Griffplatte oder als ein sich in radialer Richtung erstreckender Fingergriff ausgebildet sein kann, oder ein besonderes Teil der Spritze, das an dem Zylinder für das Zusammenwirken mit der Dosierhilfe ausgebildet ist.

Gemäß einem anderen vorteilhaften Merkmal kann zum genauen und reproduzierbaren Festlegen des mit der Spritze zu applizierenden Volumens die Dosierhilfe derart ausgebildet sein, dass sie mit der Kolbenstange verbindbar ist, insbesondere an einem proximalen Bereich oder einem axial mittigen Bereich der Kolbenstange. Im erstgenannten Fall kann ein proximaler Bereich der Dosierhilfe, insbesondere des Distanzstücks und/oder des Teilstücks, den Anschlag für den Kolben bilden, während im letztgenannten Fall ein distaler Bereich der Dosierhilfe, insbesondere des Distanzstücks, den Anschlag für den Kolben bilden kann. Weitere Vorteile der Verbindbarkeit der Dosierhilfe mit der Kolbenstange sind, dass eine Beschädigung oder gar Zerstörung des Spritzenkörpers vermieden werden kann, dass die Funktion des Spritzenkörpers nicht eingeschränkt wird und schließlich, dass weiterhin die Möglichkeit einer visuellen Kontrolle gegeben ist.

In vorteilhaften Ausführungsformen kann vorgesehen sein, dass das Distanzstück einen Längsschlitz zur Aufnahme der Kolbenstange aufweist, der insbesondere aus radialer Richtung über die Kolbenstange schiebbar und der insbesondere in radialer Richtung von der Kolbenstange abnehmbar ist. Das Verbinden der Dosierhilfe mit der Spritze und das Abnehmen der Dosierhilfe von der Spritze können aus radialer Richtung erfolgen, d. h., die Dosierhilfe wird dabei nicht axial in distaler Richtung auf die Kolbenstange aufgeschoben bzw. in proximaler Richtung von der Kolbenstange abgezogen. Dadurch ist eine besonders einfache und schnelle Verbindungsmöglichkeit der Dosierhilfe mit der Spritze geschaffen.

Ein anderes vorteilhaftes Merkmal kann sein, dass das Distanzstück und/oder das Teilstück im radialen Querschnitt teilkreisförmig ausgebildet ist. Auch in diesem Fall kann das Verbinden der Dosierhilfe mit der Spritze und das Abnehmen der Dosierhilfe von der Spritze aus radialer Richtung erfolgen, d. h., die Dosierhilfe kann dabei durch eine nicht axiale Richtung mit der Kolbenstange verbindbar sein. Das Distanzstück und/oder das Teilstück kann sich im radialen Querschnitt über einen Winkelbereich von mindestens 10° erstrecken, insbesondere zwischen 25° und 310°, vorzugsweise zwischen 30° und 300°, weiter vorzugsweise zwischen 170° und 300°.

Ein weiteres vorteilhaftes Merkmal kann darin bestehen, dass die Dosierhilfe derart elastisch ausgebildet ist, dass sie manuell aufspreizbar ist, wobei die Dosierhilfe insbesondere in radialer Richtung auf die Spritze aufsetzbar ist und/oder in radialer Richtung von der Spritze abziehbar ist. In einem auf die Spritze aufgesetzten, jedoch nicht aufgespreizten Zustand, kann die Dosierhilfe form- und/oder kraftschlüssig mit der Spritze verbindbar, insbesondere verbunden, sein. Die Dosierhilfe kann in diesem Fall kraftschlüssig durch die elastische Spannung und/oder formschlüssig an der Spritze befestigbar, insbesondere mit dieser befestigt sein.

Gemäß einer vorteilhaften weiteren Ausgestaltung kann dabei vorgesehen sein, dass die Dosierhilfe mindestens einen, insbesondere mindestens zwei, sich in radialer Richtung erstreckenden Fingerspreizhebelgriff aufweist, wobei die Dosierhilfe mittels des mindestens einen Fingerspreizhebelgriffs insbesondere beim Verbinden der Dosierhilfe mit der Spritze und/oder deren Entfernen von der Spritze manuell aufspreizbar ist. Der Fingerspreizhebelgriff bietet dabei nicht nur eine Angriffsfläche für die Finger, um die Dosierhilfe aufzuspreizen, sondern verringert aufgrund der radialen Erstreckung und der damit verbundenen Hebelwirkung die zum Aufspreizen erforderliche Fingerkraft. Um die Handhabung des Fingerspreizhebelgriffs zu verbessern, kann eine den Fingern zugewandte Oberfläche profiliert oder geriffelt sein.

Eine Sollbruchstelle im Sinne der Erfindung ist eine durch eine besondere Struktur, Gestalt oder Konstruktion (meist eine Materialverjüngung) bestimmte Stelle, die bei manueller Belastung oder Überlast vorhersagbar bricht. Insofern kann die Sollbruchstelle zum Beispiel eine Kerbe, eine Perforation, eine Ritzspur oder eine andere Verjüngung im Material sein. Durch die Kerbwirkung ist das Bauteil entscheidend geschwächt, sodass es bei Belastung oder Überlast vorhersagbar an dieser Stelle bricht. Durch die Sollbruchstelle ist das Teilstück beispielsweise durch Abbrechen, Abknicken oder Abdrehen abbrechbar, so dass die Dosierhilfe manuell axial gekürzt werden kann, um auf diese Weise verschiedene, den verkürzten Abständen entsprechende Volumen zu injizieren.

Gemäß einem vorteilhaften Merkmal umfasst die Dosierhilfe eine oder mehrere Sollbruchstellen, die als Kerbe, Ritzung, Schlitz oder Nut in der Oberfläche des Distanzstücks und/oder des Teilstücks und/oder dazwischen ausgebildet sein können. Eine solche Kerbe, Ritzung, Schlitz oder Nut kann auf dem Distanzstück und/oder dem Teilstück vollumfänglich umlaufend ausgebildet sein oder nur auf einem Teilumfang des Distanzstücks und/oder des Teilstücks ausgebildet sein.

Gemäß einem weiteren vorteilhaften Merkmal wird vorgeschlagen, dass mindestens eine Sollbruchstelle, insbesondere sämtliche Sollbruchstellen, als mehrere nebeneinander in einem Luftspalt angeordnete und den Luftspalt überbrückende, durchbrechbare Verbindungsstege ausgebildet sind. Der Luftspalt kann zwischen dem Distanzstück und dem Teilstück und/oder zwischen zwei axial benachbart angeordneten Teilstücken ausgebildet sein. Vorzugsweise weist das Distanzstück und/oder das Teilstück an derartigen Sollbruchstellen eine Art Perforation auf.

In vorteilhaften Ausgestaltungen kann die Dosierhilfe zwischen einer und zehn, bevorzugt zwischen zwei und acht und besonders bevorzugt zwischen drei und sechs Sollbruchstellen aufweisen. Das mindestens eine Teilstück kann dann in einer durch die Anzahl und die Lage der Sollbruchstellen gegebenen Staffelung des mittels der Dosierhilfe vorbestimmt einstellbaren Volumens an der gewünschten Sollbruchstelle abgebrochen und mit einer Spritze verwendet werden. Die Staffelung des einstellbaren Volumens und die entsprechende Anordnung der Sollbruchstellen an dem Teilstück kann in gleichmäßigen und/oder ungleichmäßigen Stufen erfolgen. In einer weiteren Ausgestaltung der Erfindung kann die Dosierhilfe wiederverwendbar sein.

Die Spritze kann als Spritze für jede medizinische Anwendung, unter anderem für eine ophthalmologische Anwendung, beispielsweise für eine intravitreale Injektion ausgebildet sein. Die Spritze kann eine gebräuchliche Standardspritze sein oder auch speziell für das Fixieren der Dosierhilfe und/oder das Zusammenwirken mit der Dosierhilfe ausgebildet sein, beispielsweise durch entsprechende Einrichtungen an dem Zylinder, der Fingerauflage, der Kolbenstange oder dem Kolbenkopf. Die Spritze kann eine Ein- oder eine Mehrwegspritze sein. Bevorzugt ist die Spritze bereits mit dem zu applizierenden Mittel befüllt. Insbesondere ist der Innenraum der Spritze sterilisiert, insbesondere thermisch sterilisiert, beispielsweise autoklaviert. Die Dosierhilfe ist vorzugsweise mit der Spritze lösbar verbunden. In einer weiteren Ausgestaltung der Erfindung ist der Kolben an einem distalen Ende mit einem Kolbenstopfen versehen, der aus einem Kunststoff, insbesondere aus einem Elastomer, gefertigt sein kann.

Die erfindungsgemäße Dosierhilfe weist sowohl eine hohe Benutzerfreundlichkeit als auch eine hohe Genauigkeit beim Einstellen eines von der Spritze abgebbaren, mittels der Dosierhilfe vorbestimmt einstellbaren Volumens einer Flüssigkeit auf. Beim Einstellen der Spritze auf eine gewünschte Dosis muss der Benutzer keine Skala auf der Spritze ablesen. Die Erfindung kommt damit sogar mit Spritzen aus, die überhaupt keine Messskala aufweisen. Das erleichtert die Bedienung und vermeidet Ablesefehler. Die gewünschte Injektionsmenge wird sehr genau eingehalten und ist schnell, einfach und sicher auf unterschiedliche, gewünschte Werte einstellbar. Insbesondere wird bei Injektionen das Risiko einer Überdosierung reduziert. Mit der erfindungsgemäßen Dosierhilfe kann die von einer Spritze abzugebende Flüssigkeitsmenge, insbesondere bei der Injektion medizinischer oder pharmazeutischer Flüssigkeiten, auf einfache und genaue Weise in Stufen eingestellt werden, indem das Teilstück an einer dem zu applizierenden Volumen entsprechenden Sollbruchstelle abgebrochen und damit die wirksame Länge der Dosierhilfe eingestellt wird.

Durch die erfindungsgemäße Dosierhilfe wird insbesondere für den Bereich der Ophthalmologie die Möglichkeit geschaffen, ohne eine Anpassung der Spritzengeometrie selbst für jeden Patienten individuell festzulegen, welches - patientenindividuelle - Volumen des zu applizierenden Mittels verabreicht werden soll, und die zum Verabreichen des jeweils individuell festgelegten Volumens benutzte Spritze auf einfache und genaue Weise mittels der erfindungsgemäße Dosierhilfe auf dieses Volumen einzustellen. Die Umrechnung zwischen dem gewünschten zu applizierenden Volumen und der hierfür einzustellenden axialen Länge der Dosierhilfe ist ohne Weiteres durch Kenntnis des Durchmessers der konkret verwendeten Spritze möglich.

Die erfindungsgemäße Dosierhilfe ermöglicht es daher insbesondere im Rahmen einer therapeutischen oder interventionellen Behandlung in der Medizin, vorzugsweise in der Augenheilkunde, insbesondere bei einer intravitrealen Injektion, die zu injizierende Dosis nicht pauschal und für alle Patienten gleich, sondern in Abhängigkeit von den jeweils individuell vorliegenden Eigenschaften des Auges festzulegen. So kann beispielsweise im Vergleich zu einem emmetropen (normalsichtigen) Auge bei einem kleineren, hyperopen (weitsichtigen) Auge aufgrund des durch die reduzierte Augenlänge kleineren Augenvolumens weniger Medikament injiziert werden, wogegen bei einem myopen (kurzsichtigen) Auge aufgrund des durch die größere Augenlänge größeren Augenvolumens mehr Medikament injiziert werden kann. Es kann also in jedem Fall ein an die anatomischen Verhältnisse individuell angepasstes Volumen appliziert werden. Dabei kann das hierfür jeweils festgelegte, von der Spritze abzugebende Volumen mittels der Dosierhilfe auf einfache und genaue Weise an der Spritze voreingestellt und dann mit der Spritze appliziert werden.

Die erfindungsgemäße Dosierhilfe kann jedoch nicht nur im Bereich der Ophthalmologie angewendet werden, sondern auch in anderen Bereichen der Medizin, insbesondere wenn mit einer Spritze ein kleines, insbesondere individuell angepasstes Volumen injiziert werden soll, beispielsweise in der Immunologie, Neurologie, Gynäkologe, Anästhesiologie, Dermatologie, Veterinärmedizin, Inneren Medizin, Chirurgie, Orthopädie oder Pädiatrie, sowie allgemein in nichtmedizinischen, technischen Bereichen, insbesondere wenn von einer Spritze ein kleines, insbesondere für den Einzelfall angepasstes Volumen abgegeben werden soll.

Weitere Vorteile und Merkmale der Erfindung ergeben sich aus den Ansprüchen und der nachfolgenden Beschreibung, in der Ausführungsbeispiele der Erfindung unter Bezugnahme auf die Zeichnungen im Einzelnen erläutert sind. Die darin beschriebenen Besonderheiten können einzeln oder in Kombination miteinander eingesetzt werden, um bevorzugte Ausgestaltungen der Erfindung zu schaffen. Gleiche oder gleich wirkende Teile werden in den Figuren mit denselben Bezugszeichen bezeichnet und gewöhnlich nur einmal beschrieben, auch wenn sie bei anderen Ausführungsformen vorteilhaft eingesetzt werden können. Es zeigen:
- Fig. 1: einen Längsschnitt durch eine gebräuchliche Spritze,
- Fig. 2: die Spritze von Fig. 1 mit Bemaßungen,
- Fig. 3: einen Längsschnitt der Spritze von Fig. 1 mit einer aufgesetzten Dosierhilfe,
- Fig. 4: eine erfindungsgemäße Dosierhilfe,
- Fig. 5: die Verwendung der Dosierhilfe von Fig. 4,
- Fig. 6: eine Ansicht einer alternativen Verwendung der Dosierhilfe von Fig. 4,
- Fig. 7: einen Längsschnitt zu Fig. 6,
- Fig. 8: die Verwendung der Dosierhilfe von Fig. 6,
- Fig. 9: eine erste Seitenansicht der Dosierhilfe von Fig. 4,
- Fig. 10: eine zweite Seitenansicht der Dosierhilfe von Fig. 4,
- Fig. 11: eine axiale Ansicht der Dosierhilfe von Fig. 4,
- Fig. 12: eine erste Seitenansicht der Dosierhilfe von Fig. 9 auf einer Spritze,
- Fig. 13: eine zweite Seitenansicht der Dosierhilfe von Fig. 9 auf einer Spritze,
- Fig. 14: eine Seitenansicht einer abgewandelten erfindungsgemäßen Dosierhilfe,
- Fig. 15: einen Längsschnitt zu Fig. 14,
- Fig. 16: das Abbrechen der Dosierhilfe von Fig. 14 in einer Seitenansicht,
- Fig. 17: eine Seitenansicht der aus Fig. 16 resultierenden Dosierhilfe,
- Fig. 18: eine Seitensicht der Dosierhilfe von Fig. 14 auf einer Spritze,
- Fig. 19: einen Längsschnitt zu Fig. 18,
- Fig. 20: eine erste Seitenansicht einer abgewandelten erfindungsgemäßen Dosierhilfe,
- Fig. 21: eine zweite Seitenansicht der Dosierhilfe von Fig. 20,
- Fig. 22: eine axiale Ansicht der Dosierhilfe von Fig. 20,
- Fig. 23: eine Seitenansicht der Dosierhilfe von Fig. 20 beim Aufsetzen auf eine Spritze,
- Fig. 24: eine erste Seitenansicht der Dosierhilfe von Fig. 20 auf einer Spritze,
- Fig. 25: eine zweite Seitenansicht der Dosierhilfe von Fig. 20 auf einer Spritze,
- Fig. 26: die Dosierhilfe von Fig. 20,
- Fig. 27: einen Längsschnitt zu Fig. 26e,
- Fig. 28: die Verwendung der Dosierhilfe von Fig. 26 und
- Fig. 29: eine alternative Verwendung der Dosierhilfe von Fig. 26.

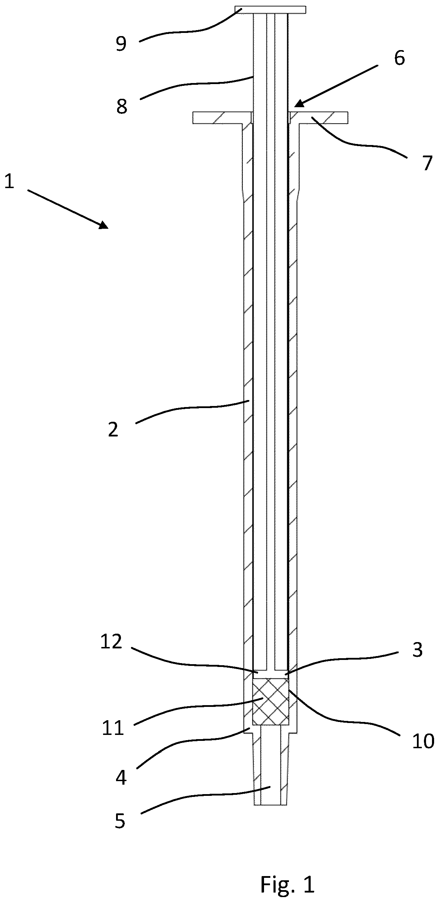

Die Fig. 1 veranschaulicht in einem Längsschnitt die Komponenten einer gebräuchlichen Spritze 1, die mit einer erfindungsgemäßen Dosierhilfe verwendet werden kann. Sie umfasst einen Zylinder 2, in dessen zylindrischem Innen- oder Hohlraum ein Kolben 3 axial beweglich ist. Der Zylinder 2 weist an dem distalen, also zu einem Patienten gerichteten Ende einen Kolbenboden 4 mit einer Düse 5 auf. Am proximalen Ende weist der Zylinder 2 eine Zylinderöffnung 6 auf und eine Fingerauflage 7, die auch Fingergriff oder Griffplatte genannt wird. Der Kolben 3 kann mittels einer Kolbenstange 8 in axialer Richtung in dem Zylinder 2 bewegt werden, wobei die Kolbenstange 8 an ihrem proximalen Ende einen Kolbenkopf 9 aufweist, der auch Daumenauflage genannt wird. Der Kolben 3 ist an seinem distalen Ende mittels einer Kolbendichtung 10, die dicht an der Innenfläche des Zylinders 2 anliegt und sich längs der Achse des Zylinders 2 frei bewegen kann, gegenüber dem Zylinder 2 flüssigkeitsdicht abgedichtet und kann hierzu beispielsweise einen Kolbenstopfen 11 aufweisen, der vorzugsweise aus einem Elastomer besteht und mittels einer Befestigungsplatte 12 oder einem Befestigungsgewinde mit der Kolbenstange 8 verbunden ist.

Der Kolben 3 ist mittels der Kolbenstange 8 in axialer Längsrichtung des Zylinders 2 nach distal und nach proximal in dem Zylinder 2 bewegbar. Wenn die Kolbenstange 8 in einer distalen Vorwärtsbewegung, das heißt von proximal nach distal bewegt wird, wozu in der Regel die Finger einer Hand benutzt werden und wobei der Daumen auf den Kolbenkopf 9 drückt und zwei andere Finger ein Widerlager an der Fingerauflage 7 finden, wird aus dem distalen Ende des Zylinders 2 ein zu applizierendes Mittel durch die Düse 5 ausgestoßen. Die Menge des applizierten Mittels bestimmt sich durch den dabei axialen Hub des Kolbens 3. In Fig. 1 ist der Kolben 3 soweit wie möglich in den Zylinder 2 hineingedrückt dargestellt, die Spritze 1 ist also entleert.

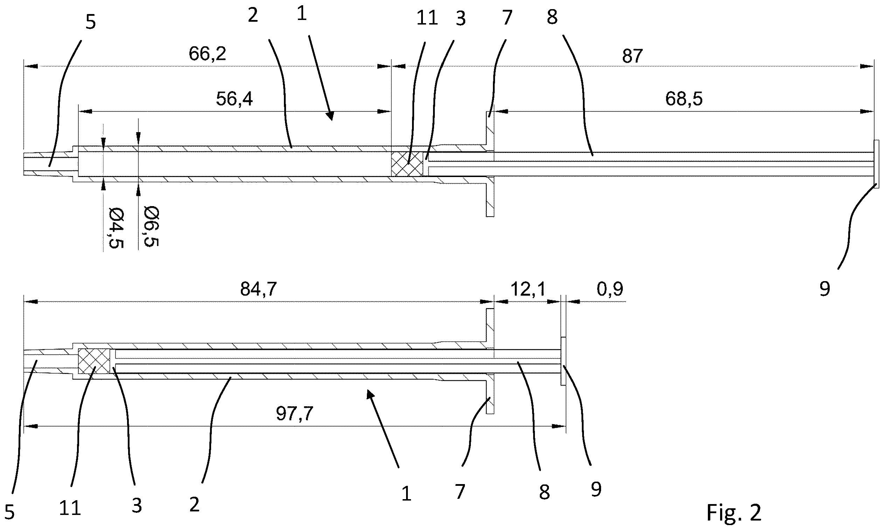

In Fig. 2 ist die Spritze 1 von Fig. 1 mit beispielshaften Abmessungen in mm dargestellt. Die Spritze 1 ist im oberen Teil von Fig. 2 mit ausgezogenem Kolben 3 und im unteren Teil von Fig. 2 mit ganz hineingedrücktem Kolben 3 dargestellt. Es handelt sich bei diesem Ausführungsbeispiel um eine Einmal-Insulinspritze des Typs Braun Omnifix 100 Solo mit einem Gesamtvolumen von 1 ml.

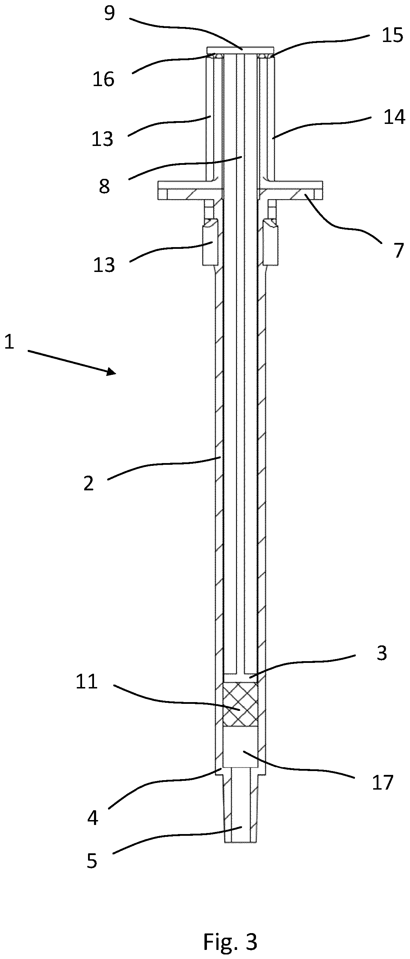

Die Fig. 3 zeigt einen Längsschnitt der Spritze 1 von Fig. 1 mit einer darauf aufgesetzten und von dem Zylinder 2 gehaltenen, erfindungsgemäßen Dosierhilfe 13, die zum Einstellen eines von der Spritze 1 abgebbaren, mittels der Dosierhilfe 13 vorbestimmt einstellbaren Volumens 17 einer Flüssigkeit als zu applizierendem Mittel vorgesehen und ausgebildet ist. Die Dosierhilfe 13 ist manuell auf die Spritze 1 aufsetzbar und manuell von der Spritze 1 abnehmbar und bildet mit ihrem nach proximal weisenden Distanzstück 14 einen den distalen Vorschub des Kolbens 3 begrenzenden Anschlag 15 für den Kolben 3. In dem Ausführungsbeispiel der Fig. 3 weist hierzu das proximale Ende der Dosierhilfe 13 eine Anschlagsfläche 16 auf, die derart ausgebildet ist, dass sie das distale Vorschieben des Kolbens 3 begrenzt, wenn das Distanzstück 14 auf die Spritze 1 aufgesetzt ist und das distale Ende eines an der Kolbenstange 8 angeordneten Kolbenkopfes 9 an der Anschlagsfläche 16 anliegt. Somit kann der Kolben 3 durch das Anliegen der Anschlagsfläche 16 an dem Kolbenkopf 9 nicht weiter in den Zylinder 2 hineingeschoben werden.

In Fig. 3 ist der Kolben 3 maximal in den Zylinder 2 eingeschoben, das heißt bis zu der Position, in der Anschlag 15 der auf die Spritze 1 aufgesetzten Dosierhilfe 13 wirksam wird und das weitere, distale Hineinschieben des Kolbens 3 verhindert. In dieser Position befindet sich in dem distalen Ende des Zylinders 2 noch immer Flüssigkeit mit einem durch die Position des Kolbens 3, die durch die axiale Länge der Dosierhilfe 13 bestimmt wird, einstellbar vorbestimmten Volumen 17. Wenn nun die Dosierhilfe 13 von der Spritze 1 manuell abgenommen wird, begrenzt die Dosierhilfe 13 nicht mehr das Hineineinschieben des Kolbens 3 in den Zylinder 2. Zum Ausstoßen der Flüssigkeit mit dem vorbestimmten Volumen 17 kann der Kolben 3 dann weiter nach distal in den Zylinder 2 hineingedrückt werden, bis er die Endposition gemäß Fig. 1 erreicht. Dabei verbleibt üblicherweise etwas Restflüssigkeit in der Düse 5 und eventuell in sehr geringem Maße auch in dem Zylinder 2. Die Differenz der Menge der von der Spritze 1 aufgenommenen Flüssigkeit in der Position, in der der Kolben 3 maximal bis zum Wirksamwerden des Anschlags 15 des auf die Spritze 1 aufgesetzten Distanzstücks 14 in den Zylinder 2 eingeschoben ist gemäß Fig. 3, zu der Menge der in der Spritze 1 verbleibenden Restflüssigkeit in der Position, in der der Kolben 3 maximal in den Zylinder 2 eingeschoben ist gemäß Fig. 1, ohne dass das Distanzstück 14 auf die Spritze 1 aufgesetzt ist, entspricht dem von der Spritze 1 abgebbaren, einstellbar vorbestimmten Volumen 17.

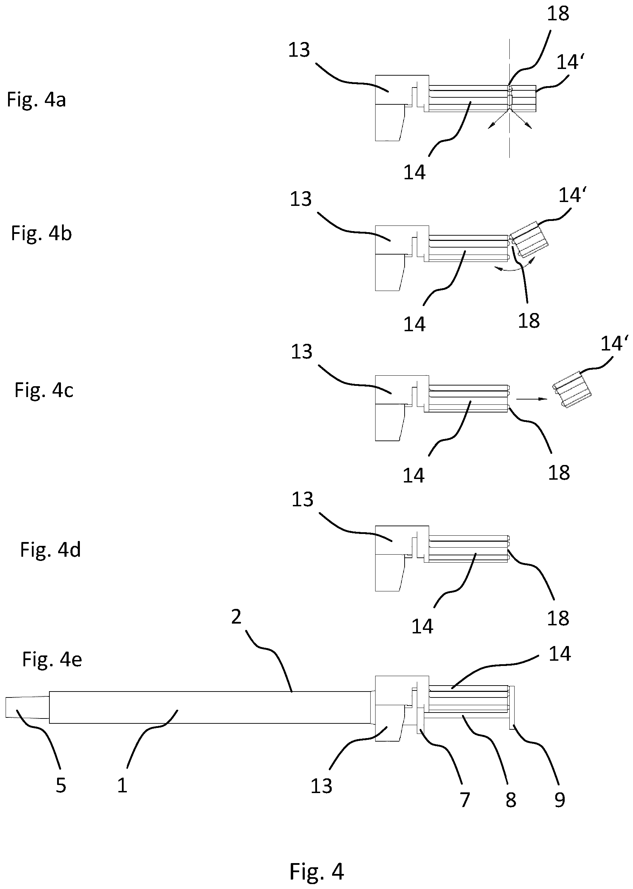

Die Fig. 4 veranschaulicht in den aufeinanderfolgenden Schritten der Fig. 4a bis 4e die Verwendung einer erfindungsgemäßen Dosierhilfe 13. Sie weist die Besonderheit auf, dass das, proximale, Distanzstück 14 der Dosierhilfe proximal über eine Sollbruchstelle 18 mit einem Teilstück 14' verbunden ist, das damit manuell brechbar ist. Damit bildet das Distanzstück 14, wie bereits im Zusammenhang mit Fig. 3 beschrieben, nach dem Abbrechen des Teilstücks 14' an der Sollbruchstelle 18 und der damit einhergehenden Verkürzung der in axialer Richtung der Spritze 1 als Anschlag wirksamen Länge der Dosierhilfe 13 einen den distalen Vorschub des Kolbens 3 begrenzenden Anschlag 15 für den Kolben 3, bei dem das zu der Position der Sollbruchstelle 18 korrespondierende, von der Spritze 1 abgebbare, vorbestimmt einstellbare Volumen 17 kleiner ist als ohne Abbrechen eines Teils des Distanzstücks 14 an der Sollbruchstelle 18 des Distanzstücks 14.

Das in Fig. 4 dargestellte Ausführungsbeispiel einer Dosierhilfe 13 umfasst eine solche Sollbruchstelle 18 mit dem dieser zugeordnetem Teilstück 14'. In Fig. 4a ist die Sollbruchstelle 18 in dem Teilstück 14' noch ungebrochen. Wird die Dosierhilfe 13 dergestalt verwendet, wird damit wie in Fig. 3 erläutert ein größeres von der Spritze 1 abgebbares Volumen 17 eingestellt werden können. Um die Dosierhilfe 17 zum Einstellen eines demgegenüber kleineren, von der Spritze 1 abgebbaren Volumens 17 verwenden zu können, wird wie in Fig. 4b veranschaulicht das Teilstück 14' an der Sollbruchstelle 18 gebrochen, wodurch sich seine axiale Länge verkürzt, und das abgebrochene Stück wie in Fig. 4c gezeigt entsorgt. Es verbleibt dann eine in Fig. 4d gezeigte Dosierhilfe 13 mit einem an der Sollbruchstelle 18 entnommenen Teilstück 14', die dann wie in Fig. 4e gezeigt auf die Spritze 1 aufgesetzt wird und wie in Fig. 3 erläutert mit der Spritze 1 zum Einstellen und Abgeben eines vorbestimmt einstellbaren Volumens 17 verwendet wird.

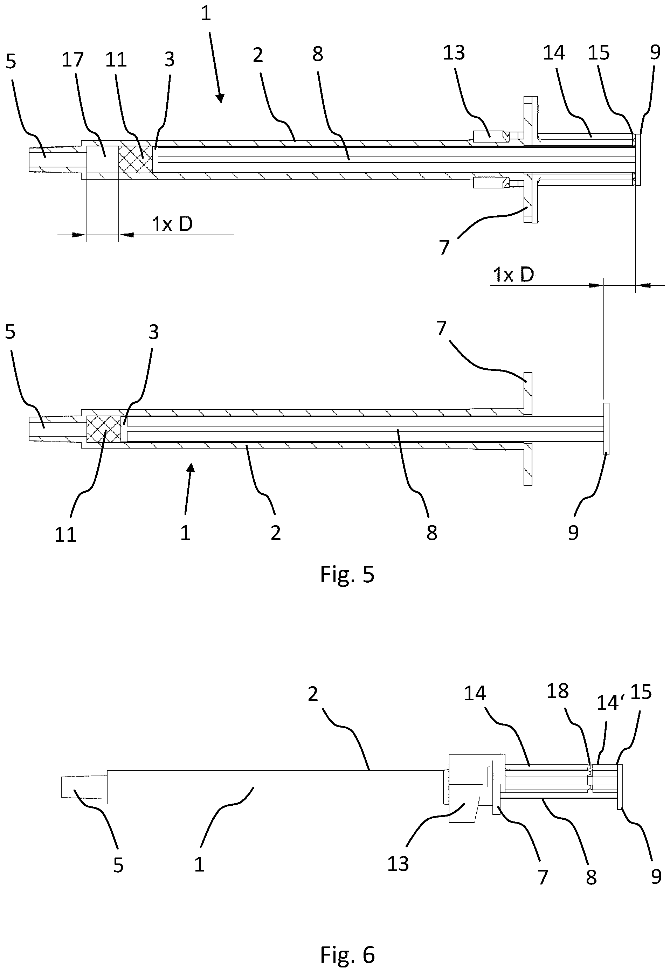

Die Verwendung der Dosierhilfe 13 von Fig. 4 wird anhand der geschnittenen Darstellung der Fig. 5 erläutert. Der obere Teil der Fig. 5 ist ein Längsschnitt zu der Fig. 4e, wenn der Kolben 3 bei auf die Spritze 1 aufgesetzter Dosierhilfe 13 maximal in den Zylinder 2 eingeschoben ist, und entspricht insoweit der Fig. 3. Wenn die Dosierhilfe 13 dann abgenommen wird, kann mit der Spritze 1 das voreingestellte Volumen 17 abgegeben werden, indem der Kolben 3 ganz in den Zylinder 2 hineingedrückt wird, wie in dem unteren Teil der Fig. 5 dargestellt, der insoweit der Fig. 1 entspricht. Das von der Spritze 1 dabei abgegebene Volumen 17 entspricht beispielsweise einer einfachen gewünschten Dosis 1x D.

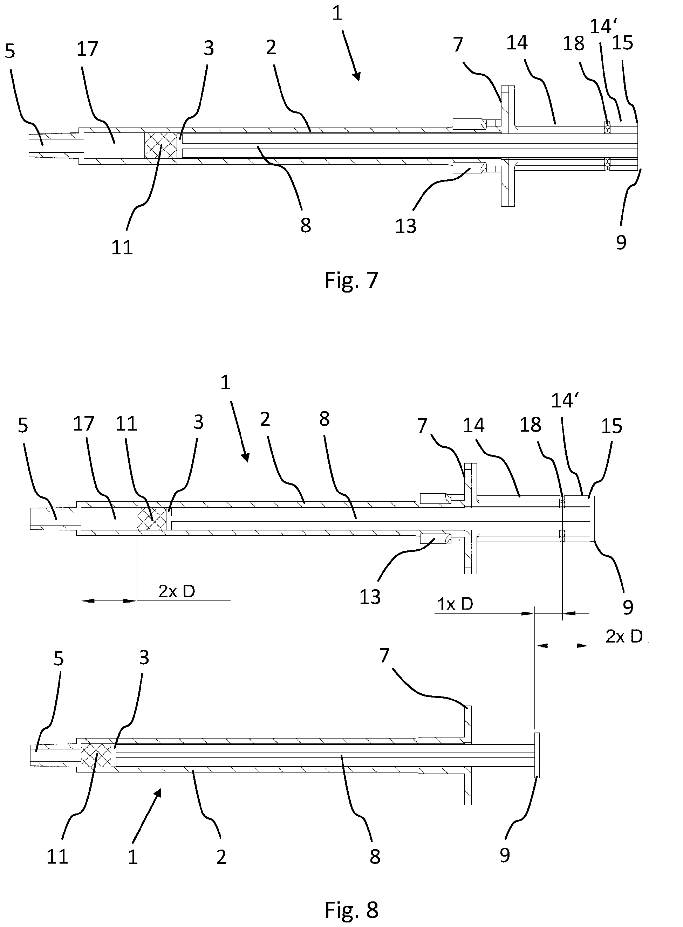

Wenn die in Fig. 4 dargestellte Dosierhilfe 13 nicht wie in Fig. 4a an der Sollbruchstelle 18 abgebrochen, sondern ungekürzt entsprechend der in Fig. 3 erläuterten Weise verwendet wird, kann sie zum Einstellen eines größeren mit der Spritze 1 abzugebenden Volumens 17 verwendet werden. Dies wird in den Fig. 6 bis 8 veranschaulicht. Die Fig. 6 zeigt eine Ansicht dieser alternativen Verwendung der Dosierhilfe 13 von Fig. 4a, wenn die Dosierhilfe 13 auf die Spritze 1 aufgesetzt und der Kolben 3 bis zum Anschlagen des Kolbenkopfes 9 an dem mit dem Distanzstück 14 noch immer verbundenen Teilstück 14' in den Zylinder 2 hineingedrückt ist. Die Fig. 6 entspricht damit in gewisser Weise der Fig. 4e mit einem längeren Distanzstück 14. Die Fig. 7 zeigt einen Längsschnitt zu Fig. 6 und entspricht der Fig. 3 mit einem längeren Distanzstück 14.

Die Verwendung der Dosierhilfe 13 von Fig. 6 und 7 zum Einstellen und Abgeben eines entsprechend größeren, vorbestimmt einstellbaren Volumens 17 wird anhand der geschnittenen Darstellung der Fig. 8 erläutert. Der obere Teil der Fig. 8 ist der Längsschnitt zu der Fig. 6 gemäß Fig. 7, wenn der Kolben 3 bei auf die Spritze 1 aufgesetzter Dosierhilfe 13 maximal in den Zylinder 2 eingeschoben ist, und entspricht insoweit näherungsweise der Fig. 3 mit einem Distanzstück 14, das länger als in Fig. 5 ist. Wenn die Dosierhilfe 13 dann abgenommen wird, kann mit der Spritze 1 das voreingestellte Volumen 17 abgegeben werden, indem der Kolben 3 ganz in den Zylinder 2 hineingedrückt wird, wie in dem unteren Teil der Fig. 8 dargestellt, der insoweit der Fig. 1 entspricht. Das von der Spritze 1 dabei abgegebene Volumen 17 entspricht beispielsweise einer gewünschten doppelten Dosis 2x D.

Die sich beim Verwenden der an der Sollbruchstelle 18 abgebrochenen Dosierhilfe 13 ergebende Dosierung einer einfachen Dosis 1x D, wie sie anhand der Fig. 5 erläutert wurde, ist in Fig. 8 zum Vergleich ebenfalls eingezeichnet, obwohl diese Dosierung von 1x D beim Verwenden der Dosierhilfe 13 in der in Fig. 8 veranschaulichten Weise nicht erfolgt. Die in der Fig. 8 benutzte Dosierhilfe 13 verbraucht sich dabei jedoch nicht und kann folglich bei weiteren Dosierungen gemäß Fig. 8 oder, wenn sie an der Sollbruchstelle 18 abgebrochen wird, bei weiteren Dosierungen gemäß Fig. 5 verwendet werden. Die erfindungsgemäße Dosierhilfe ist damit wiederverwendbar.

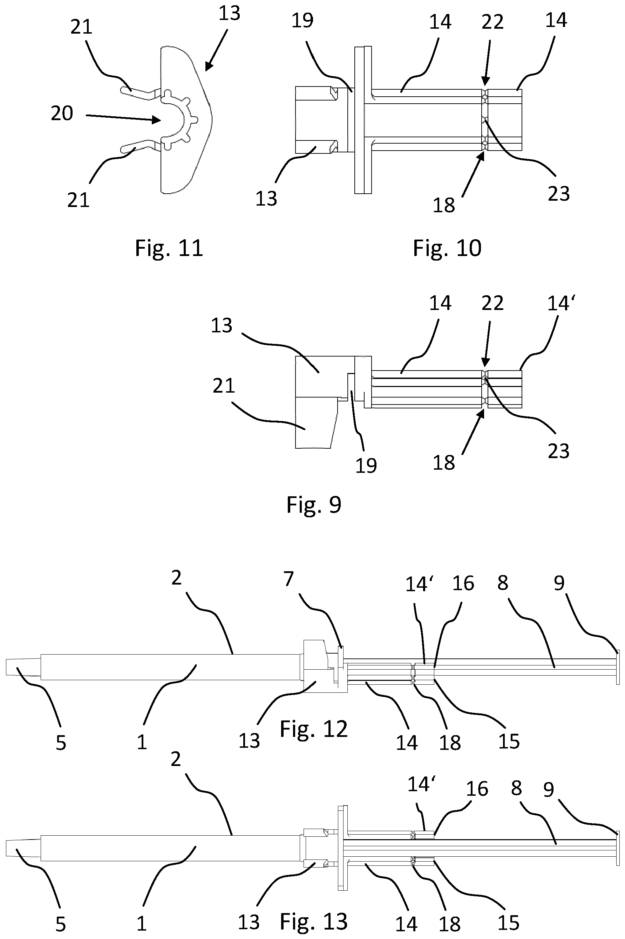

Die Fig. 9 bis 11 zeigen weitere Einzelheiten der erfindungsgemäßen Dosierhilfe 13 von Fig. 4a. Die Fig. 9 ist eine erste, radiale Seitenansicht der Dosierhilfe 13, die Fig. 10 eine Aufsicht der Dosierhilfe 13 und die Fig. 11 eine axiale Ansicht der Dosierhilfe 13 von Fig. 4a, die derart ausgebildet ist, dass sie an dem Zylinder 2 lösbar verbindbar ist. Hierzu Sie weist sie eine teilkreisförmige Vertiefung 19 oder einen teilkreisförmigen Schlitz auf, die bzw. der auf einen radialen Vorsprung am proximalen Ende des Zylinders 2 aufsteckbar ist, wobei das Distanzstück 14 in einer vordefinierten axialen Relativposition mit der Spritze 1 verbunden ist. Die teilkreisförmige Vertiefung 19 ist eine Art Ringnut, die einen radialen Vorsprung an dem Zylinder 2, beispielsweise die Fingerauflage 7, aufnehmen kann, um dadurch die Dosierhilfe 13, insbesondere das Distanzstück 14 mit dem damit über die Sollbruchstelle 18 verbundenen Teilstück 14', in einer vordefinierten axialen Relativposition mit der Spritze 1 zu verbinden. Das Distanzstück 14 und das Teilstück 14' weisen jeweils einen Längsschlitz 20 auf, der aus radialer Richtung über die Kolbenstange 8 geschoben und in radialer Richtung von der Kolbenstange 8 abgenommen werden kann. Das Distanzstück 14 ist im radialen Querschnitt teilkreisförmig ausgebildet und erstreckt sich über einen Winkelbereich von etwa 220°.

Die Dosierhilfe 13 ist derart elastisch ausgebildet, dass sie manuell aufspreizbar ist, um sie aus radialer Richtung auf die Spritze 1 aufzusetzen oder aus radialer Richtung von der Spritze 1 abzuziehen, und wenn sie auf die Spritze 1 aufgesetzt und nicht aufgespreizt ist, form- und/oder kraftschlüssig mit der Spritze 1 verbunden ist. Um das Aufspreizen, Aufsetzen oder Abziehen zu erleichtern, weist die Dosierhilfe 13 sich in radialer Richtung erstreckende Fingerspreizhebelgriffe 21 auf, mittels derer sie beim Aufsetzen auf die Spritze 1 oder beim Abnehmen von der Spritze 1 manuell aufspreizbar ist. Ferner weist die Dosierhilfe 13 im unteren Bereich zwei Vorsprünge auf, die sich elastisch verformen, um den Zylinder 2 klemmend zu umschließen. Die Sollbruchstelle 18 kann beispielsweise als Kerbe, Ritzung, Schlitz oder Nut in der Oberfläche des Distanzstücks 14 ausgebildet sein. In dem in Fig. 9 und 10 dargestellten Ausführungsbeispiel ist die Sollbruchstelle 18 als mehrere nebeneinander in einem Luftspalt 22 zwischen dem Distanzstück 14 und dem Teilstück 14' angeordnete und den Luftspalt 22 überbrückende, durchbrechbare Verbindungsstege 23 ausgebildet.

Die Fig. 12 zeigt eine radiale Seitenansicht und die Fig. 13 eine Aufsicht der Dosierhilfe 13 von Fig. 9, wenn sie auf den Zylinder 2 einer Spritze 1 aufgesteckt ist. Im Unterschied zu Fig. 6 ist dabei der Kolben 3 nicht maximal bis zum Wirksamwerden des Anschlags 15 durch Anliegen des Kolbenkopfes 9 an dem Anschlag 15 hineingeschoben, sondern der Kolben 3 ist weiter aus dem Zylinder 2 herausgezogen dargestellt. Eine solche Situation ergibt sich beispielsweise, wenn die Spritze 1 vorbereitend mit Flüssigkeit aufgezogen wird, und in dieser Position ist noch kein Abgeben eines mittels des Distanzstücks 14 einstellbar vorbestimmten Volumens 17 mit der Spritze 1 möglich. Hierzu muss der Kolben 3 erst bis zu der durch das Distanzstück 14 und dessen Anschlag 15 definierten Tiefe in die in Fig. 3 beschriebene Ausgangsstellung in den Zylinder 2 hineingedrückt werden.

Das in den Fig. 4, 6 und 9 dargestellte Ausführungsbeispiel der Dosierhilfe 13 weist eine Sollbruchstelle 18 auf, sodass damit zwei verschiedene, vorbestimmt einstellbare Volumen 17 realisierbar sind, je nachdem, ob das Teilstück 14' an der Sollbruchstelle 18 gebrochen wird oder nicht. Um mittels einer Dosierhilfe 13 mehr als zwei verschiedene Volumen 17 vorbestimmt einstellen zu können, kann die Dosierhilfe 13 mehrere, beispielsweise zwischen einem und zehn, bevorzugt zwischen zwei und acht und besonders bevorzugt zwischen drei und sechs Sollbruchstellen 18 aufweisen. Damit einhergehend kann die Dosierhilfe 13 mehrere, axial hintereinander angeordnete und jeweils über Sollbruchstellen 18 verbundene Teilstücke 14' aufweisen. Die Teilstücke 14' können dann in einer durch die Anzahl und die Lage der Sollbruchstellen 18 gegebenen Staffelung des mittels der Dosierhilfe 13 vorbestimmt einstellbaren Volumens 17 an der gewünschten Sollbruchstelle 18 abgebrochen und mit einer Spritze 1 verwendet werden. Die Staffelung des einstellbaren Volumens 17 kann in gleichmäßigen und/oder ungleichmäßigen Stufen erfolgen. Bei einer gleichmäßigen Staffelung kann das ein- oder mehrfache einer Dosis eingestellt werden, bei einer ungleichmäßigen Staffelung beispielsweise eine Dosis in voller Höhe (bei maximaler Länge der ungekürzten Dosierhilfe 13) sowie um das ein- oder mehrfache eines Teilbetrages der Dosis (bei entsprechenden axialen Längen der Teilstücke 14') reduziert, beispielsweise um jeweils 10 % oder 20 %.

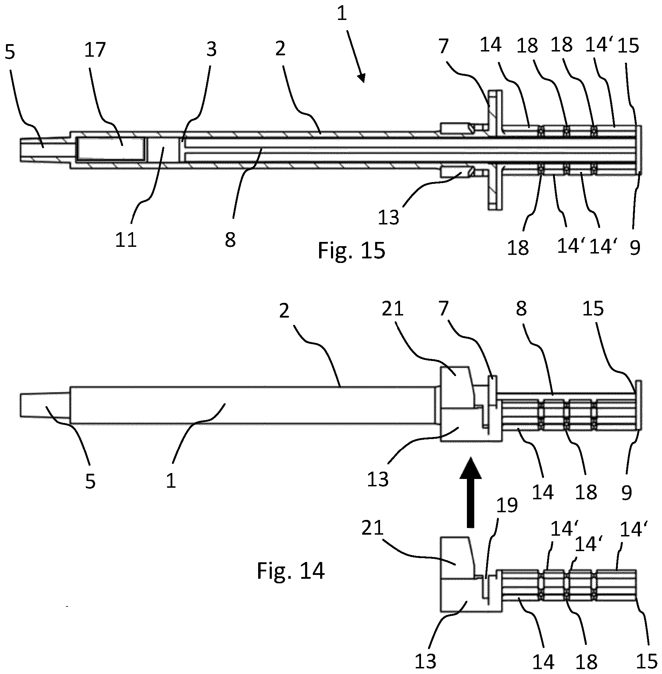

Die Fig. 14 und 15 zeigen ein abgewandeltes Ausführungsbeispiel einer Dosierhilfe 13, im unteren Teil der Fig. 14 in einer Seitenansicht und im oberen Teil der Fig. 14 in einer Seitenansicht nach dem Aufsetzen auf den Spritzenkolben 2 der Spritze 1 mit distal bis zum Anschlag durch das Distanzstück 14 eingeschobenen Kolben 3 und in Fig. 15 in einem Längsschnitt. Die Dosierhilfe 13 entspricht im Wesentlichen der Dosierhilfe 13 von Fig. 4, 6 und 9 und weist im Unterschied dazu nicht nur eine, sondern drei axial hintereinander angeordnete, jeweils über Sollbruchstellen 18 miteinander verbundene Teilstücke 14' auf, so dass die Dosierhilfe 13 durch Abbrechen an einer der Sollbruchstellen 18 auf insgesamt vier unterschiedlich Längen eingestellt werden kann, wodurch sich vier verschiedene, zugehörige mit der Spritze 1 abzugebende Volumen 17 bzw. Dosen einstellen lassen.

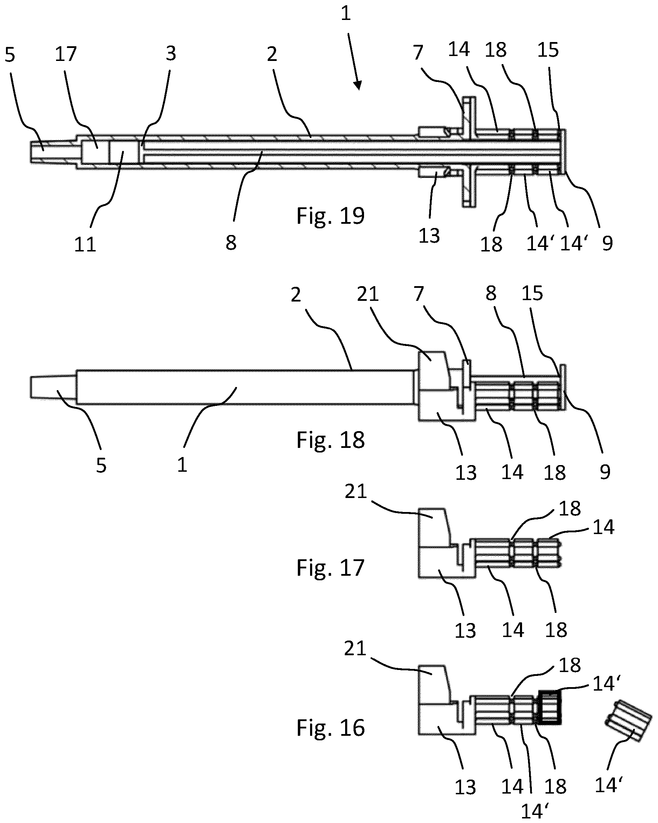

In den Fig. 14 und 15 wird die Dosierhilfe 13 von Fig. 14 ungekürzt, das heißt ohne abgebrochenes Teilstück 14' an einer der Sollbruchstellen 18 verwendet. Die Fig. 16 bis 19 veranschaulichen die Verwendung der Dosierhilfe 13 von Fig. 14, wenn das proximale Teilstück 14' zum Einstellen eines kleineren Volumens 17 an der Sollbruchstelle 18, die am nächsten zu dem Kolbenkopf 9 liegt, abgebrochen wird. Die Fig. 16 zeigt das Abbrechen der Dosierhilfe 13 von Fig. 14 in einer Seitenansicht, die Fig. 17 eine Seitenansicht der aus Fig. 16 resultierenden Dosierhilfe 13 mit abgebrochenen Teilstück 14', die Fig. 18 eine Seitensicht der Dosierhilfe 13 von Fig. 14 auf einer Spritze 1 mit bis zum Anschlag durch das Distanzstück 14 eingeschobenem Kolben 3 und die Fig. 19 einen Längsschnitt zu Fig. 18. Entsprechend kann die Dosierhilfe 13 von Fig. 14 auch verwendet werden, wenn mehrere Teilstücke 14' an einer der anderen Sollbruchstellen 18 abgebrochen werden, um ein noch kleineres Volumen 17 einzustellen.

Eine Dosierhilfe 13 mit zwei Teilstücken 14' und folglich zwei Sollbruchstellen 18 kann damit auf drei verschiedene Längen und somit auf drei entsprechende zu dosierende verschiedene Volumen 17 eingestellt werden. Sie kann beispielsweise wie folgt mit der in Fig. 2 dargestellten Einwegspritze Braun Omnifix 100 Solo zum Applizieren des Medikaments Bevacizumab in den Glaskörper eines Auges verwendet werden. Das Volumen des menschlichen Auges liegt zwischen 3 ml und 9 ml. Für eine möglichst gleiche Wirkstoffgabe können insgesamt drei sinnvolle Gruppen definiert werden: Gruppe 1 (zwischen 3 ml und 5 ml des Glaskörpers) mit einer Wirkstoffgabe von 1,25 mg (= 50 µl Bevacizumab) und damit einer Länge des Distanzstücks 14 von 14,4 mm, Gruppe 2 (zwischen 6 ml und 7 ml) mit einer Wirkstoffgabe von 1,88 mg (= 75 µl Bevacizumab) mit einer Länge des Distanzstücks 14 von 15,8 mm und Gruppe 3 (zwischen 8 ml und 9 ml) mit einer Wirkstoffgabe von 2,5 mg (= 100 µl Bevacizumab) mit einer Länge des Distanzstücks 14 von 17,2 mm. Zum Verabreichen des jeweils damit eingestellten Volumens 17 muss die Dosierhilfe 14 von der Kolbenstange 8 bzw. von dem Zylinder 2 abgezogen werden. Die Spritze 1, beispielsweise eine Spritze 1 für eine intravitreale Injektion, und die Dosierhilfe 13 bilden ein aufeinander abgestimmtes Set zum Verabreichen eines vorbestimmten Volumens 17, in dem beschriebenen Beispiel zum Applizieren des Medikaments Bevacizumab.

Bei den in den Fig. 3 bis 19 dargestellten Ausführungsbeispielen von Dosierhilfen 13 handelt es sich um Dosierhilfen 13, die mit dem Zylinder 2 der Spritze 1 verbindbar sind. Hierzu kann die Dosierhilfe 13 wie erläutert eine teilkreisförmige Vertiefung 19 oder einen teilkreisförmigen Schlitz aufweisen, die bzw. der auf einen radialen Vorsprung am proximalen Ende des Zylinders 2 aufsteckbar ist, vorzugsweise auf die Fingerauflage 7. Dadurch wird die Dosierhilfe, insbesondere das Distanzstück 14 und das mit diesem verbundene Teilstück 14', in einer vordefinierten axialen Relativposition mit der Spritze 1 verbunden.

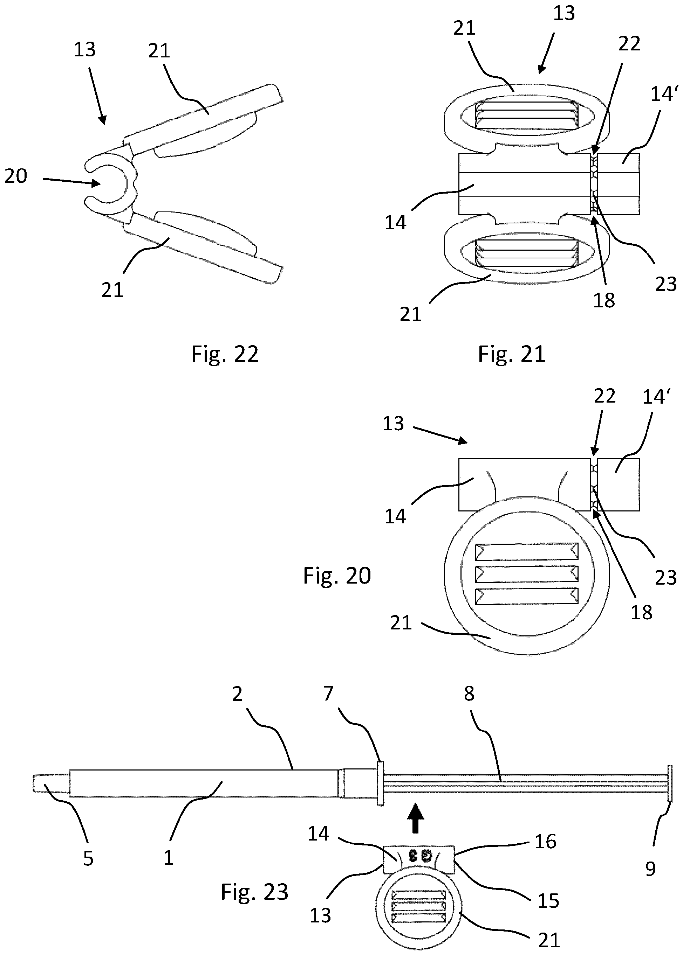

Bei den in den Fig. 20 bis 29 dargestellten Ausführungsbeispielen von Dosierhilfen 13 handelt es sich um Dosierhilfen 13, die mit der Kolbenstange 8 der Spritze 1 verbindbar sind und keine teilkreisförmige Vertiefung 19 oder einen teilkreisförmigen Schlitz aufweisen, die bzw. der auf einen radialen Vorsprung am proximalen Ende des Zylinders 2 aufsteckbar ist. Die weitere Ausgestaltung der Dosierhilfe 13 entspricht vorzugsweise den in den Fig. 3 bis 19 gezeigten Varianten, beispielsweise hinsichtlich einer Anschlagsfläche 16 am proximalen Ende des Distanzstücks 14 und des Teilstücks 14' der Dosierhilfe 13, eines Längsschlitzes 20, der aus radialer Richtung über die Kolbenstange 8 geschoben und in radialer Richtung von der Kolbenstange 8 abgenommen werden kann und im radialen Querschnitt teilkreisförmig ausgebildet ist, einer elastischen Ausbildung der Dosierhilfe 13, sodass sie manuell aufspreizbar ist, um sie aus radialer Richtung auf die Spritze 1 aufzusetzen oder aus radialer Richtung von der Spritze 1 abzuziehen, und wenn sie auf die Spritze 1 aufgesetzt und nicht aufgespreizt ist, form- und/oder kraftschlüssig an der Spritze 1 befestigt ist, sich in radialer Richtung erstreckender Fingerspreizhebelgriffe 21, mittels derer die Dosierhilfe 13 beim Aufsetzen auf die Spritze 1 oder beim Abnehmen von der Spritze 1 manuell aufspreizbar ist, Sollbruchstellen 18, die als Kerbe, Ritzung, Schlitz oder Nut zwischen dem Distanzstück 14 und dem Teilstück 14' ausgebildet sind, Sollbruchstellen 18, die als mehrere nebeneinander in einem Luftspalt 22 zwischen dem Distanzstück 14 und dem Teilstück 14', oder aber auch zwischen zwei benachbarten Teilstücken 14', angeordnete und den Luftspalt 22 überbrückende, durchbrechbare Verbindungsstege 23 ausgebildet sind, und/oder der Anzahl der Sollbruchstellen 18 und/oder der Teilstücke 14'.

Die Fig. 20 bis 22 zeigen Einzelheiten einer abgewandelten erfindungsgemäßen Dosierhilfe 13, die mit der Kolbenstange 8 verbindbar ist. Die Fig. 20 ist eine radiale Seitenansicht der Dosierhilfe 13, die Fig. 21 eine Aufsicht auf die Dosierhilfe 13 und die Fig. 22 eine axiale Ansicht der Dosierhilfe 13. Das Distanzstück 14 und das Teilstück 14' weisen jeweils einen Längsschlitz 20 auf, der aus radialer Richtung über die Kolbenstange 8 geschoben und in radialer Richtung von der Kolbenstange 8 abgenommen werden kann. Das Distanzstück 14 und das Teilstück 14' sind jeweils im radialen Querschnitt teilkreisförmig ausgebildet und erstrecken sich über einen Winkelbereich von etwa 290°.

Die Dosierhilfe 13 ist derart elastisch ausgebildet, dass sie manuell aufspreizbar ist, um sie aus radialer Richtung auf die Spritze 1 aufzusetzen oder aus radialer Richtung von der Spritze 1 abzuziehen, und wenn sie auf die Spritze 1 aufgesetzt und nicht aufgespreizt ist, form- und/oder kraftschlüssig an der Spritze 1 befestigt ist. Um das Aufspreizen, Aufsetzen oder Abziehen zu erleichtern, weist die Dosierhilfe 13 sich in radialer Richtung erstreckende Fingerspreizhebelgriffe 21 auf, mittels derer sie beim Aufsetzen auf die Spritze 1 oder beim Abnehmen von der Spritze 1 manuell aufspreizbar ist. Die Fingerspreizhebelgriffe 21 sind zwei flügelartige Fortsätze zum Erweitern der Klemmbacken der Dosierhilfe 13, sodass die Dosierhilfe 13 auf der Kolbenstange 8 angebracht bzw. wieder von dieser abgenommen werden kann. Beim Anbringen der Dosierhilfe 13 an der Kolbenstange 8 wird die Kolbenstange 8 von der Dosierhilfe 13 so umschlossen, dass die Kolbenstange nicht von der Dosierhilfe 13 festgeklemmt wird und sich somit axial bewegen kann.

Die Fig. 23 zeigt eine radiale Seitenansicht der Dosierhilfe 13 von Fig. 20 beim Aufsetzen auf eine Spritze 1, wobei das Teilstück 14' zuvor an der Sollbruchstelle 18 wie bereits beschrieben abgebrochen wurde. Der Kolben 3 ist dabei nicht maximal bis zum Wirksamwerden des Anschlags 15 durch Anliegen des Kolbenkopfes 9 an dem Anschlag 15 hineingeschoben, sondern der Kolben 3 ist weiter aus dem Zylinder 2 herausgezogen. Eine solche Situation ergibt sich beispielsweise, wenn die Spritze 1 vorbereitend mit Flüssigkeit aufgezogen wird, und in dieser Position ist noch kein Abgeben eines mittels des Distanzstücks 14 einstellbar vorbestimmten Volumens 17 mit der Spritze 1 möglich. Hierzu muss der Kolben 3 erst bis zu der durch das Distanzstück 14 und dessen Anschlag 15 definierten Tiefe in den Zylinder 2 hineingedrückt werden.

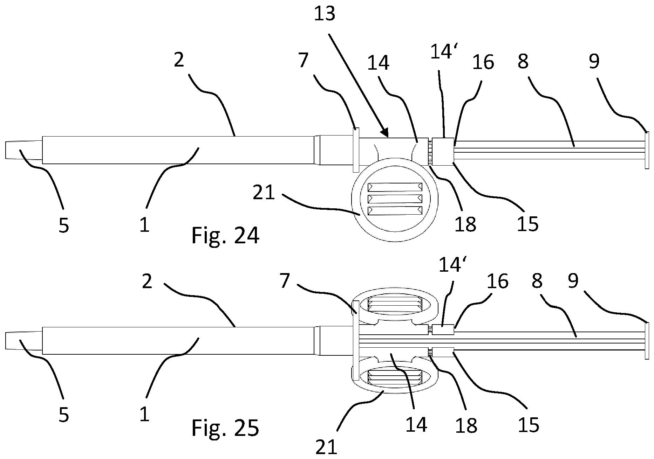

Die Fig. 24 zeigt eine radiale Seitenansicht und die Fig. 25 eine Aufsicht der Dosierhilfe 13 von Fig. 20, wenn sie auf den Zylinder 2 einer Spritze 1 aufgesteckt ist. Im Unterschied zu Fig. 23 ist dabei das Teilstück 14' nicht zuvor an der Sollbruchstelle 18 abgebrochen und somit zum Einstellen und Verabreichen eines größeren voreingestellten Volumens 17 ausgebildet.

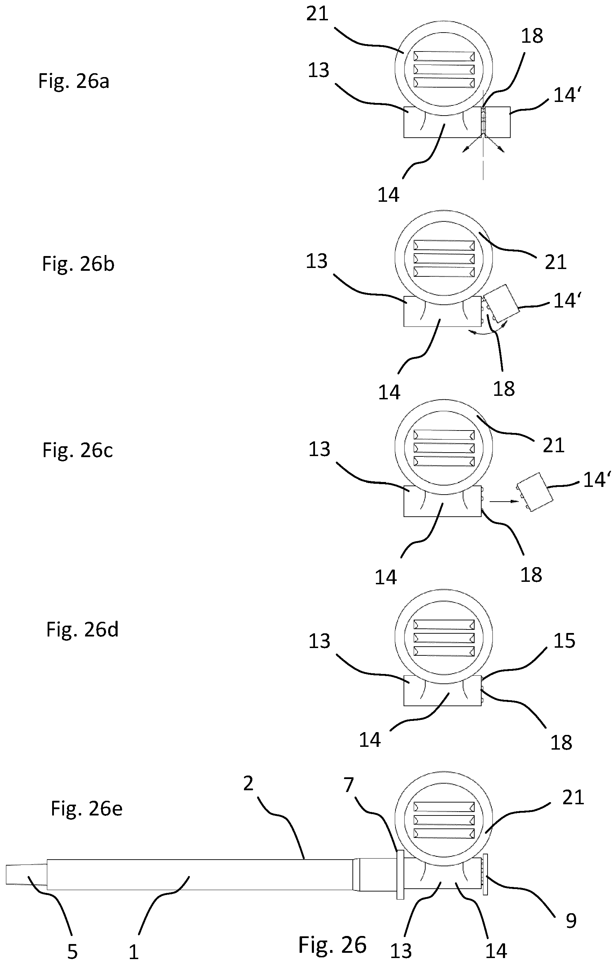

Die Fig. 26 veranschaulicht in den aufeinanderfolgenden Schritten der Fig. 26a bis 26e die Verwendung einer erfindungsgemäßen Dosierhilfe 13 von Fig. 20. Die Dosierhilfe 13 weist mindestens eine Sollbruchstelle 18 auf, an der das Teilstück 14' manuell abbrechbar ist, so dass das nach proximal weisende Distanzstück 14 wie bereits beschrieben einen den distalen Vorschub des Kolbens 3 begrenzenden Anschlag 15 für den Kolben 3 bildet, bei dem das zu der Position der Sollbruchstelle 18 korrespondierende, von der Spritze 1 abgebbare, vorbestimmt einstellbare Volumen 17 kleiner ist als ohne Abbrechen des Teilstücks 14'.

Das in Fig. 26a dargestellte Ausführungsbeispiel einer Dosierhilfe 13 umfasst genau eine solche Sollbruchstelle 18 und genau ein Teilstück 14'. In Fig. 26a ist die Sollbruchstelle 18 noch ungebrochen und das Teilstück 14' über diese mit dem Distanzstück 14 verbunden. Wenn die Dosierhilfe 13 so verwendet würde, würde damit wie in Fig. 24 und 25 gezeigt ein größeres von der Spritze 1 abgebbares Volumen 17 eingestellt werden können. Um die Dosierhilfe 17 zum Einstellen eines demgegenüber kleineren, von der Spritze 1 abgebbaren Volumens 17 verwenden zu können, wird wie in Fig. 26b veranschaulicht das Teilstück 14' an der Sollbruchstelle 18 gebrochen, wodurch sich die axiale Länge der Dosierhilfe 13 verkürzt, und das abgebrochene Teilstück 14' wie in Fig. 26c gezeigt entsorgt. Es verbleibt dann eine in Fig. 26d gezeigte Dosierhilfe 13 mit dem Distanzstück 14, die dann wie in Fig. 26e gezeigt auf die Spritze 1 aufgesetzt wird und wie in Fig. 23 dargestellt zum Einstellen und Abgeben eines vorbestimmt einstellbaren Volumens 17 mit einer Spritze 1 verwendet wird.

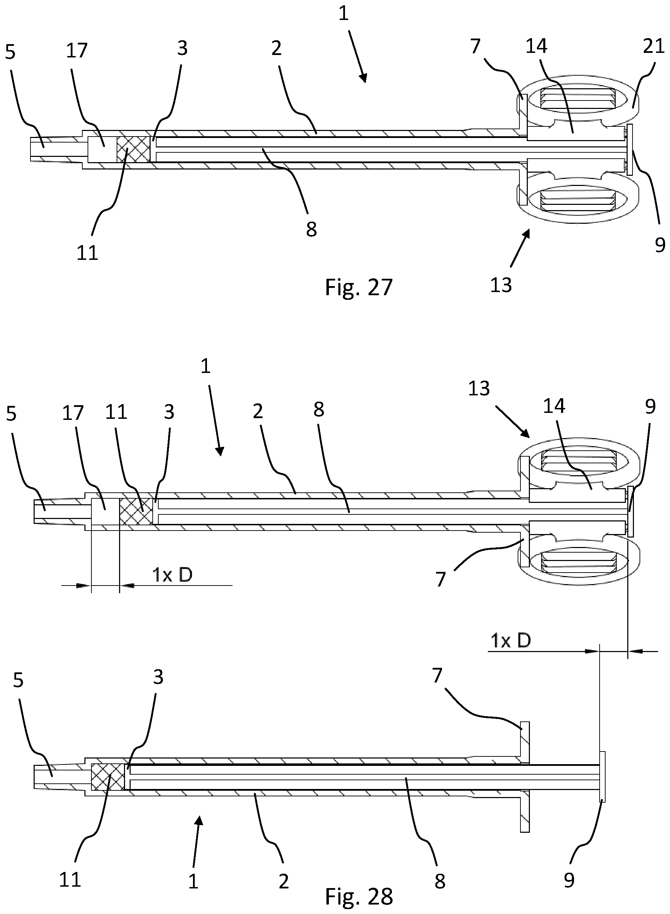

Die Fig. 27 zeigt einen Längsschnitt zu Fig. 26e. Die Verwendung der Dosierhilfe 13 von Fig. 26 wird anhand des Längsschnitts der Fig. 28 erläutert. Der obere Teil der Fig. 28 ist ein Längsschnitt zu der Fig. 26e, wenn der Kolben 3 bei auf die Spritze 1 aufgesetzter Dosierhilfe 13 maximal in den Zylinder 2 eingeschoben ist. Wenn die Dosierhilfe 13 dann abgenommen wird, kann mit der Spritze 1 das voreingestellte Volumen 17 abgegeben werden, indem der Kolben 3 ganz nach distal in den Zylinder 2 hineingedrückt wird, wie in dem unteren Teil der Fig. 28 dargestellt. Das von der Spritze 1 dabei abgegebene Volumen 17 entspricht beispielsweise einer einfachen gewünschten Dosis 1x D.

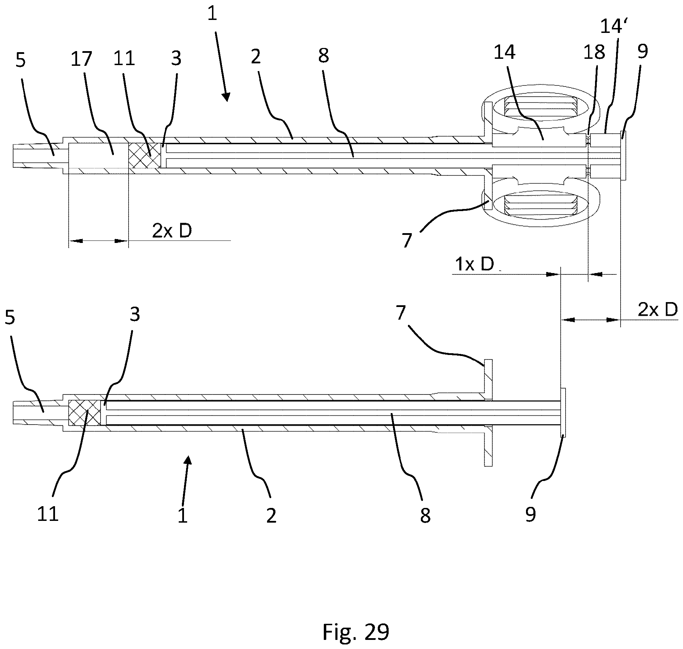

Die Verwendung der Dosierhilfe 13 von Fig. 20, 24, 25 und 26a zum Einstellen und Abgeben eines entsprechend größeren, vorbestimmt einstellbaren Volumens 17 wird anhand der geschnittenen Darstellung der Fig. 29 erläutert. Der obere Teil der Fig. 29 zeigt einen Längsschnitt, wenn der Kolben 3 bei auf die Spritze 1 aufgesetzter Dosierhilfe 13 maximal in den Zylinder 2 eingeschoben ist, und entspricht insoweit der Fig. 3 mit einem Distanzstück 14, das jedoch länger als in Fig. 28 ist. Wenn die Dosierhilfe 13 dann abgenommen wird, kann mit der Spritze 1 das voreingestellte Volumen 17 abgegeben werden, indem der Kolben 3 ganz nach distal in den Zylinder 2 hineingedrückt wird, wie in dem unteren Teil der Fig. 29 dargestellt. Das von der Spritze 1 dabei abgegebene Volumen 17 entspricht beispielsweise einer gewünschten doppelten Dosis 2x D.

Die sich beim Verwenden der an der Sollbruchstelle 18 abgebrochenen Dosierhilfe 13 ergebende Dosierung einer einfachen Dosis 1x D, wie sie anhand der Fig. 28 erläutert wurde, ist in Fig. 29 zum Vergleich ebenfalls eingezeichnet, obwohl diese Dosierung von 1x D beim Verwenden der Dosierhilfe 13 in der in Fig. 29 veranschaulichten Weise nicht erfolgt. Die in der Fig. 29 benutzte Dosierhilfe 13 verbraucht sich dabei jedoch nicht und kann folglich bei weiteren Dosierungen gemäß Fig. 29 oder, wenn sie an der Sollbruchstelle 18 abgebrochen wird, bei weiteren Dosierungen gemäß Fig. 28 verwendet werden.

## Patentansprüche

1. Dosierhilfe (13) für eine Spritze (1), die einen Zylinder (2) und einen Kolben (3) umfasst, der mittels einer Kolbenstange (8) in dem Zylinder (2) axial beweglich ist, wobei die Dosierhilfe (13) mit der Spritze (1) lösbar verbindbar ist, wobei die Dosierhilfe (13) in einem proximalen Bereich mindestens ein sich nach proximal erstreckendes Distanzstück (14) aufweist, das einem Anschlag (15) für den Kolben (3) entspricht, wobei das Distanzstück (14) über eine Sollbruchstelle (18) mit einem Teilstück (14') derart verbunden ist, dass das Teilstück (14') manuell abbrechbar ist.

2. Dosierhilfe (13) nach Anspruch 1, **dadurch gekennzeichnet, dass** die Dosierhilfe (13) mindestens zwei axial hintereinander angeordnete Teilstücke (14') aufweist, die jeweils über Sollbruchstellen (18) miteinander derart verbunden sind, dass die Teilstücke (14) manuell abbrechbar sind.

3. Dosierhilfe (13) nach einem der vorhergehenden Ansprüche, **dadurch gekennzeichnet, dass** die Dosierhilfe (13) derart ausgebildet ist, dass sie mit dem Zylinder (2) der Spritze (1) verbindbar ist.

4. Dosierhilfe (13) nach Anspruch 3, **dadurch gekennzeichnet, dass** die Dosierhilfe (13) eine teilkreisförmige Vertiefung (19) aufweist, die auf einen radialen Vorsprung insbesondere am proximalen Ende des Zylinders (2) aufsteckbar ist, wobei insbesondere das Distanzstück (14) und/oder das mindestens eine Teilstück (14') in einer vordefinierten axialen Relativposition mit der Spritze (1) anordenbar ist.

5. Dosierhilfe (13) nach einem der vorhergehenden Ansprüche , **dadurch gekennzeichnet, dass** die Dosierhilfe (13) derart ausgebildet ist, dass sie mit der Kolbenstange (8) verbindbar ist.

6. Dosierhilfe (13) nach einem der vorhergehenden Ansprüche, **dadurch gekennzeichnet, dass** das Distanzstück (14) einen Längsschlitz (20) zur Aufnahme der Kolbenstange (8) aufweist, der insbesondere aus radialer Richtung über die Kolbenstange (8) schiebbar und der insbesondere in radialer Richtung von der Kolbenstange (8) abnehmbar ist.

7. Dosierhilfe (13) nach einem der vorhergehenden Ansprüche, **dadurch gekennzeichnet, dass** das Distanzstück (14) und/oder das Teilstück (14') im radialen Querschnitt teilkreisförmig ausgebildet ist.

8. Dosierhilfe (13) nach einem der vorhergehenden Ansprüche, **dadurch gekennzeichnet, dass** sie derart elastisch ausgebildet ist, dass sie manuell aufspreizbar ist, wobei die Dosierhilfe (13) insbesondere in radialer Richtung auf die Spritze (1) aufsetzbar ist und/oder in radialer Richtung von der Spritze (1) abziehbar ist.

9. Dosierhilfe (13) nach Anspruch 8, **dadurch gekennzeichnet, dass** die Dosierhilfe (13) mindestens einen, insbesondere mindestens zwei, sich in radialer Richtung erstreckenden Fingerspreizhebelgriff (21) aufweist, wobei die Dosierhilfe (13) mittels des mindestens einen Fingerspreizhebelgriffs (21) insbesondere beim Verbinden der Dosierhilfe (13) mit der Spritze (1) und/oder deren Entfernen von der Spritze (1) manuell aufspreizbar ist.

10. Dosierhilfe (13) nach einem der vorhergehenden Ansprüche, **dadurch gekennzeichnet, dass** die Sollbruchstelle (18), insbesondere sämtliche Sollbruchstellen (18) als Kerbe, Ritzung, Schlitz oder Nut in der Oberfläche des Distanzstücks (14) ausgebildet ist.

11. Dosierhilfe (13) nach einem der vorhergehenden Ansprüche, **dadurch gekennzeichnet, dass** die mindestens eine Sollbruchstelle (18), insbesondere sämtliche Sollbruchstellen (18) als mehrere nebeneinander in einem Luftspalt (22) angeordnete und den Luftspalt (22) überbrückende, durchbrechbare Verbindungsstege (23) ausgebildet sind.

12. Set umfassend eine Spritze (1), insbesondere eine Spritze (1) für eine intravitreale Injektion, und eine Dosierhilfe (13) nach einem der vorhergehenden Ansprüche, wobei die Dosierhilfe (13) mit der Spritze (1) lösbar verbindbar, insbesondere lösbar verbunden, ist.

13. Set nach Anspruch 12, **dadurch gekennzeichnet, dass** die Spritze (1) mit der damit verbundenen Dosierhilfe (13) zur Abgabe von mindestens zwei vordefinierter Dosierungsvolumina ausgebildet ist, wobei der axiale Hub des Kolbens (3) bis zu dem dem Teilstück (14') zugeordneten Anschlag (15) einem ersten Dosierungsvolumen entspricht und wobei der axiale Hub des dem Teilstück (14') zugeordneten Anschlag (15) bis zu dem dem Distanzstück (14) zugeordneten Anschlag (15) einem zweiten Dosierungsvolumen entspricht.
